# EUROPEAN PATENT APPLICATION

(11) **EP 4 512 409 A1**
(43) Date of publication of application: **26.02.2025**
(21) Application number: 23774022.0
(22) Date of filing: 24.03.2023
(51) Int. Cl.: A61K 36/9068, A61K 36/8888, A61K 36/75, A61K 36/71, A61K 36/634, A61K 36/539, A61K 36/28, A61K 36/233, A61K 36/232, A61K 36/076, A61P 31/22

(54) **TRADITIONAL CHINESE MEDICINE COMPOSITION FOR TREATING HERPES ZOSTER, AND PREPARATION METHOD THEREFOR**

(30) Priority: 25.03.2022 CN 202210306360
(71) Applicant: Beijing Supreme Pharmaceutical Technology Co., Ltd, Beijing 100005 (CN)
(72) Inventor: HU, Tianbao, Beijing 100005 (CN); HE, Zongyuan, Beijing 100005 (CN); SONG, Jin, Beijing 100005 (CN)
(74) Representative: Winter, Brandl - Partnerschaft mbB
(86) International application number: PCT/CN2023/083805
(87) International publication number: WO 2023/179779

(57) **Abstract**

The present disclosure provides a traditional Chinese medicine composition for treating herpes zoster and a preparation method thereof, and relates to the technical field of traditional Chinese medicine compositions. The traditional Chinese medicine composition includes the following components by weight: 20-40 portions of radix bupleuri, 30-50 portions of radix scutellariae, 10-20 portions of rhizoma pinelliae preparata, 30-50 portions of radix paeoniae rubra, 20-40 portions of stir-fried fructus aurantii, 20-40 portions of radix rhapontici, 20-40 portions of fructus forsythiae, 50-70 portions of poria cum radix pini, 10-30 portions of radix angelicae sinensis and 40-60 portions of rhizoma zingiberis recens. The present disclosure reasonably controls the content ratio of specific components of each traditional Chinese medicine and optimizes the preparation method, so that the obtained traditional Chinese medicine composition has a better therapeutic effect on herpes zoster.

## Description

### Technical field

The present disclosure relates to the technical field of traditional Chinese medicine compositions, and in particular to a traditional Chinese medicine composition for treating herpes zoster and a preparation method thereof.

### Background Art

Herpes zoster (HZ) is caused by the reactivation of varicella-zoster virus (VZV). It is a common infectious skin disease with the main clinical features of erythema, blisters or papules distributed in a band-like manner along a unilateral peripheral nerve, and often accompanied by obvious neuralgia. After the herpes subsides, some viruses still lurk in the ganglia, which causes pain in the invaded area. It is called postherpetic neuralgia. The pain can range from mild to extreme, and the pain is continuous, intermittent or induced by very small stimulation. As a stubborn disease in dermatology, postherpetic neuralgia brings great physical pain and psychological burden to patients, which seriously affects their quality of life. The treatment goals of herpes zoster are to relieve acute pain, limit the spread of skin lesions, shorten the duration of skin lesions, and prevent or alleviate postherpetic neuralgia and its other acute or chronic complications.

Currently, there are two treatments for herpes zoster: traditional Chinese medicine treatment and Western medicine treatment. Western medicine treatment is divided into antiviral treatment, glucocorticoid therapy, local treatment of neuralgia, physical therapy, treatment of postherpetic neuralgia, treatment of cranial nerve involvement, treatment of herpes zoster in children and adolescents, treatment of herpes zoster during pregnancy, treatment of herpes zoster in immunocompromised patients, and treatment of herpes zoster resistant to antiviral drugs. Commonly used anti-herpetic virus drugs include acyclovir, valacyclovir, famciclovir, and large doses of glucocorticoids. Patients with postherpetic neuralgia who experience persistent and severe pain often need to take tricyclic antidepressants, antiepileptic drugs, narcotic analgesics, etc. But these drugs may cause systemic adverse reactions and can only relieve moderate pain. Traditional Chinese medicine treatment of postherpetic neuralgia mostly adopts syndrome differentiation treatment, one is to promote blood circulation and remove blood stasis, the second is to eliminate pathogen and dredge collaterals, and the third is to strengthen vital qi. The drugs used include tranquilizers, insect-derived drugs for dredging collaterals, and drugs targeting concurrent symptoms and sites of disease.

For example, the Chinese patent application 201410762480.5 discloses a traditional Chinese medicine composition for treating herpes zoster, the raw materials of which are as follows: 25-35 portions of gypsum fibrosum, 25-35 portions of herba taraxaci, 25-35 portions of fructus forsythiae, 10-15 portions of radix scutellariae, 10-15 portions of radix rhapontici, 5-10 portions of schizonepeta tenuifolia, 5-10 portions of saposhnikovia divaricata, 5-10 portions of angelica dahurica, 5-10 portions of rhizoma et radix notopterygii, 10-15 portions of lygodium japonicum, 13-18 portions of talcum, 18-23 portions of senecio scandens, 5-10 portions of cicada slough, 10g of herba spirodelae, 5-10 portions of radix bupleuri, 10-15 portions of paeonia lactiflora, 5-10 portions of cortex moutan, 5-10 portions of gleditsia sinensis, 10-15 portions of endothelium corneum gigeriae galli and 5-10 portions of platycodon grandiflorum. This composition can be taken orally or applied externally for treating herpes zoster and has the characteristics of rapid onset of action, good efficacy and high cure rate.

Another example is the Chinese patent application No. 201310303546.X which discloses a traditional Chinese medicine composition taken orally for treating herpes zoster. This Chinese medicine composition is composed of the following raw material medicine proportions by weight: 24-26g of fructus arctii, 18-22g of kochiae fructus, 18-22g of cicada slough, 10-12g of radix scrophulariae, 18-22g of radix isatidis, 10-12g of radix scutellariae and 14-16g of rhizoma menispermi. This traditional Chinese medicine has reasonable formula, low cost, convenient drug material acquisition, simple production, short treatment course, significant efficacy, high cure rate, no toxic side effects, and is not prone to recurrence.

There is a compatibility relationship between traditional Chinese medicine compositions, so that the medicines are arranged into a "hierarchy", according to roles of the "monarch", the "minister", the "underlings" and the "envoy", and then integrated to make the medicines become a whole, so as to achieve a better therapeutic effect. However, the existing traditional Chinese medicine compositions have poor compatibility effects, and there are problems such as long treatment courses and slow effects. Therefore, it is necessary to develop a traditional Chinese medicine composition for treating herpes zoster with good compatibility effects and excellent therapeutic effects and a preparation method thereof.

### Summary

In view of the problems existing in the prior art, the present disclosure provides a traditional Chinese medicine composition for treating herpes zoster with good compatibility and therapeutic effect and a preparation method thereof, which is based on the theory of traditional Chinese medicine and controls the specific components and content ratios of traditional Chinese medicine..

In order to achieve the above-mentioned purpose, the present disclosure provides a traditional Chinese medicine composition for treating herpes zoster, comprising the following components: radix bupleuri, radix scutellariae, rhizoma pinelliae preparata, radix paeoniae rubra, stir-fried fructus aurantii, radix rhapontici, fructus forsythiae, poria cum radix pini, radix angelicae sinensis and rhizoma zingiberis recens.

In particular, the weight ratio of the radix bupleuri, stir-fried fructus aurantii, radix rhapontici and fructus forsythiae is 0.5-2:0.5-2:1:1; preferably, the weight ratio of the radix bupleuri, stir-fried fructus aurantii, radix rhapontici and fructus forsythiae is 1-2:0.5-2:1:1; further preferably, the weight ratio of the radix bupleuri, stir-fried fructus aurantii, radix rhapontici and fructus forsythiae is 1:1:1:1.

The weight ratio of the radix scutellariae, poria cum radix pini and radix angelicae sinensis is 1-5:2-7:1; preferably, the weight ratio of the radix scutellariae, poria cum radix pini and radix angelicae sinensis is 2-4:3-5:1; further preferably, the weight ratio of the radix scutellariae, poria cum radix pini and radix angelicae sinensis is 2:3:1.

The traditional Chinese medicine composition comprises the following components by weight: 20-40 portions of radix bupleuri, 30-50 portions of radix scutellariae, 10-20 portions of rhizoma pinelliae preparata, 30-50 portions of radix paeoniae rubra, 20-40 portions of stir-fried fructus aurantii, 20-40 portions of radix rhapontici, 20-40 portions of fructus forsythiae, 50-70 portions of poria cum radix pini, 10-30 portions of radix angelicae sinensis and 40-60 portions of rhizoma zingiberis recens.

Preferably, the traditional Chinese medicine composition comprises the following components by weight: 25-35 portions of radix bupleuri, 35-45 portions of radix scutellariae, 12-18 portions of rhizoma pinelliae preparata, 35-45 portions of radix paeoniae rubra, 25-35 portions of stir-fried fructus aurantii, 25-35 portions of radix rhapontici, 25-35 portions of fructus forsythiae, 55-65 portions of poria cum radix pini, 15-25 portions of radix angelicae sinensis and 45-55 portions of rhizoma zingiberis recens.

Further preferably, the traditional Chinese medicine composition comprises the following components by weight: 30 portions of radix bupleuri, 40 portions of radix scutellariae, 15 portions of rhizoma pinelliae preparata, 40 portions of radix paeoniae rubra, 30 portions of stir-fried fructus aurantii, 30 portions of radix rhapontici, 30 portions of fructus forsythiae, 60 portions of poria cum radix pini, 20 portions of radix angelicae sinensis and 40 portions of rhizoma zingiberis recens.

After long-term research, it was found that patients with herpes zoster have some common characteristics: at first, the disease is caused by hidden pathogen. Secondly, it occurs usually on one side of the body. Thirdly, the disease occurs when there are some unexpected changes in the body, which is usually called a decrease in resistance. Fourthly, the symptoms of refreshing are basically the same, mainly pain and blisters. It is said in the Shanghan Lun that "when the blood is weak and the qi is exhausted, the pores open and the pathogen enters the body and accumulates under the ribs". Thus, from the perspective of classical prescriptions, herpes zoster belongs to Shaoyang disease.

Therefore, based on the theory of traditional Chinese medicine, a traditional Chinese medicine composition that can effectively treat herpes zoster and relieve pain is produced by mixing radix bupleuri, radix scutellariae, rhizoma pinelliae preparata, radix paeoniae rubra, stir-fried fructus aurantii, radix rhapontici, fructus forsythiae, poria cum radix pini, radix angelicae sinensis and rhizoma zingiberis recens. In the composition, the radix bupleuri is used as the "monarch" medicine, and is combined with the radix scutellariae as the "minister" medicine to harmonize and clear the heat, so as to eliminate the pathogen of Shaoyang. Peony softens the liver, relieves acute pain and stops pain. When combined with fructus aurantii, the peony can regulate qi and promote blood circulation to eliminate the pain of epigastric lumpy stiffness. Pinellia harmonizes the stomach and descends the adverse qi. It is combined with a large amount of rhizoma zingiberis recens together as a "underlings" medicine to treat continuous vomiting; the radix rhapontici and fructus forsythiae have the effects of clearing heat and detoxifying, eliminating carbuncles and dispersing nodules. When combined with radix angelicae sinensis, they enter the blood and can nourish blood, promote blood circulation and stop pain. The three herbs are also as an "underlings" medicine. Patients with herpes zoster usually suffer from unbearable pain, irritability, anxiety and insomnia. Therefore, Poria cum radix pini is used as an "underlings" medicine in the prescription to calm the mind, soothe the nerves and promote diuresis. Rhizoma zingiberis recens can harmonize the nutrient and defense and circulate body fluids, and it can also regulate the spleen and stomach, and it has multiple functions as the roles of "underlings", and "envoy".

The properties, taste, channel tropism and effects of each herb in the traditional Chinese medicine composition of the present disclosure are as follows:
Radix bupleuri: slightly cold in nature, bitter and pungent in taste, enters the liver meridian and gallbladder meridian, has the effects of soothing the liver and benefiting the gallbladder, soothing qi and relieving depression, and dispersing fire; indications: liver depression and qi stagnation, chest and rib swelling pain, rectal prolapse, uterine shedding, and irregular menstruation.
Radix scutellariae: cold in nature, bitter in taste, enters the lung meridian, gallbladder meridian, spleen meridian, large intestine meridian and small intestine meridian; has the effects of clearing heat, eliminating dampness, purging fire, detoxifying, stopping bleeding and preventing miscarriage.
Rhizoma pinelliae preparata: warm in nature, spicy in taste, enters the spleen meridian, stomach meridian and lung meridian. It has the effects of eliminating dampness and resolving phlegm. Indications: It is used for dry throat, cough and asthma due to excessive phlegm, dizziness and palpitations due to phlegm and fluid, dizziness due to wind-phlegm, and headache due to phlegm syncope.
Radix paeoniae rubra: bitter in taste, slightly cold in nature. It enters the liver meridian. It has the effects of clearing heat, cooling blood, dispersing blood stasis and relieving pain. Indications: treats amenorrhea due to blood stasis, accumulation of hernia, abdominal pain, rib pain, epistaxis, bloody diarrhea, intestinal bleeding, red eyes, carbuncle swelling, and injuries from falls.
Stir-fried fructus aurantii: bitter in taste, slightly sour, enters spleen meridian and stomach meridian. It has the effects of regulating qi, relaxing the middle energizer, removing stagnation and relieving bloating. Indications: qi stagnation in the chest and ribs, fullness and pain, indigestion, phlegm retention; gastroptosis, rectal prolapse, uterine prolapse.
Radix rhapontici: bitter in taste, cold in nature; enters the stomach meridian; has the effects of clearing heat and detoxifying, eliminating carbuncles and dispersing nodules, promoting menstruation and lactation, relaxing tendons and unblocking blood vessels. Indications: Swelling and pain caused by breast carbuncles, scrofula sore, retention of breast milk, damp bi and cramps.
Fructus forsythiae: bitter in taste, slightly cold in nature; enters the lung meridian, heart meridian, and small intestine meridian; has the effects of clearing heat and detoxifying, reducing swelling and dispersing nodules, and evacuating wind-heat. Indications: sores, carbuncles, swellings and scrofula, phlegm and nodules, wind-heat exogenous infection, initial stage of febrile disease, hot and painful stranguria.
Poria cum radix pini: sweet and light in taste, neutral in nature; enters the heart meridian and spleen meridian; has the effects of calming the mind, soothing the nerves, and promoting diuresis. Indications: palpitations, forgetfulness, insomnia, epilepsy.
Radix angelicae sinensis: sweet and spicy in taste, warm in nature; enters the liver meridian, heart meridian, and spleen meridian; has the effects of nourishing blood and regulating menstruation, promoting blood circulation and relieving pain, and moistening the intestines and promoting bowel movements. Indications: blood deficiency and sallow, irregular menstruation, amenorrhea and dysmenorrhea, blood deficiency, blood stagnation, blood cold, traumatic injury, rheumatism and bi-pain, carbuncle and ulcer, intestinal dryness and constipation, etc.
Rhizoma zingiberis recens: spicy in taste and warm in nature; enters the lung meridian, spleen meridian, and stomach meridian; has the effects of relieving exterior syndrome, dispelling cold, warming the spleen and stomach and stopping vomiting, warming the lungs and stopping cough, and detoxifying. Indications: Wind-cold colds, cold spleen and stomach syndrome, stomach cold vomiting, lung cold cough, and can also detoxify fish and crabs.

The present disclosure also provides a preparation method for the above-mentioned traditional Chinese medicine composition, including the following steps:
(1) Weighing a prescribed amount of radix bupleuri, stir-fried fructus aurantii, radix rhapontici and fructus forsythiae, adding water and boiling to obtain a residue 1 and a filtrate 1;
(2) Mixing the residue 1 with radix scutellariae, poria cum radix pini and radix angelicae sinensis, adding water and boiling to obtain a filtrate 2;
(3) Mixing the filtrate 1 and the filtrate 2 and concentrating to obtain the traditional Chinese medicine composition.

Before the boiling in the step (1), add water and soak the herbs in water for 20-40 minutes, preferably 30 minutes. The boiling time is 30-50 minutes; preferably 40 minutes, the boiling is repeated 1-2 times; the ratio of the added water volume to the total mass of radix bupleuri, stir-fried fructus aurantii, radix rhapontici and fructus forsythiae is 6-10:1 mL/g, preferably 8:1 mL/g.

The boiling time in above step (2) is 1-1.5 hours; preferably 1.5 hours; the boiling is repeated 1-2 times, the ratio of the added water volume to the total mass of residue 1, radix scutellariae, poria cum radix pini and radix angelicae sinensis is 5-8: 1mL/g, preferably 6:1 mL/g.

In some preferred embodiments, the filtrate 1 is also added in above step (2), and the volume ratio of the filtrate 1 to the added water is 1-3:10, preferably 2:10.

During the implementation of the present disclosure, it was unexpectedly found that adding a certain amount of filtrate 1 to the boiling liquid during the boiling process can promote the dissolution of the active ingredients of the herbs and improve the therapeutic effect of the medicine composition.

The dosage form of the traditional Chinese medicine composition of the present disclosure can be traditional Chinese medicine decoction, pills, powders, or tablets, granules, or capsules in modern pharmaceutical dosage forms.

The present disclosure also provides a use of the above traditional Chinese medicine composition in the production of a drug for treating herpes zoster.

Compared with the existing technology, the beneficial effects of the disclosure are that:
(1) the traditional Chinese medicine composition provided in the present disclosure includes the components of radix bupleuri, radix scutellariae, rhizoma pinelliae preparata, radix paeoniae rubra, stir-fried fructus aurantii, radix rhapontici, fructus forsythiae, poria cum radix pini, radix angelicae sinensis and rhizoma zingiberis recens, in particular, the radix bupleuri is used as the "monarch" medicine, and is combined with the radix scutellariae as the "minister" medicine to harmonize and clear the heat, so as to eliminate the pathogen of Shaoyang. Peony softens the liver, relieves acute pain and stops pain. When combined with fructus aurantii, the peony can regulate qi and promote blood circulation to eliminate the pain of epigastric lumpy stiffness. Pinellia harmonizes the stomach and descend the adverse qi. It is combined with a large amount of rhizoma zingiberis recens together as a "underlings" medicine to treat continuous vomiting; the radix rhapontici and fructus forsythiae have the effects of clearing heat and detoxifying, eliminating carbuncles and dispersing nodules. When combined with radix angelicae sinensis, they enter the blood and can replenish blood, promote blood circulation and stop pain. The three herbs are also as an "underlings" medicine. Patients with herpes zoster usually suffer from unbearable pain, irritability, anxiety and insomnia. Therefore, Poria cum radix pini is used as an "underlings" medicine in the prescription to calm the mind, soothe the nerves and promote diuresis. Rhizoma zingiberis recens can harmonize the nutrient and defense and circulate body fluids, and it can also regulate the spleen and stomach, and it has multiple functions as the roles of "underlings", and "envoy". Through the interaction between each component, the disclosure obviously improves the drug treatment effect.
(2) The traditional Chinese medicine composition provided by the present disclosure has good efficacy in treating herpes zoster, and has the advantages of good efficacy, rapid effect, short treatment time and few side effects. Clinical observations have shown that, the efficacy of the traditional Chinese medicine composition of the present disclosure can reach 100%.

### BRIEF DESCRIPTION OF THE DRAWINGS

FIG. 1 is a comparison diagram of the effects of case 1 before and after medication in a specific embodiment of the present application;
FIG.2 is a comparison diagram of the effects of case 2 before and after medication in a specific embodiment of the present application.

### DETAILED DESCRIPTION

The disclosure is further elaborated in detail below in conjunction with specific embodiment, and following embodiment is not used to limit the disclosure, is only used to illustrate the disclosure. Unless otherwise specified, the experimental methods used in the following embodiment, for which specific conditions are not specified in the embodiment are generally carried out under conventional conditions. Unless otherwise specified, the materials, reagents, etc. used in the following embodiment are all commercially available.

The present disclosure is further described below in conjunction with specific embodiments. These embodiments are only used to illustrate the present disclosure and are not intended to limit the scope of the present disclosure.

### Example 1 A traditional Chinese medicine composition for treating herpes zoster and preparation method thereof

The Chinese medicine composition includes the following components by weight: 20 portions of radix bupleuri, 30 portions of radix scutellariae, 10 portions of rhizoma pinelliae preparata, 30 portions of radix paeoniae rubra, 30 portions of stir-fried fructus aurantii, 20 portions of radix rhapontici, 20 portions of fructus forsythiae, 50 portions of poria cum radix pini, 10 portions of radix angelicae sinensis and 40 portions of rhizoma zingiberis recens.
preparation method:
(1) Weighing a prescribed amount of radix bupleuri, stir-fried fructus aurantii, radix rhapontici and fructus forsythiae, adding water and soaking 20 minutes, then boiling 30 minutes and 2 times to obtain a residue 1 and a filtrate 1;
(2) Mixing the residue 1 with radix scutellariae, poria cum radix pini and radix angelicae sinensis, adding water and boiling 1h to obtain a filtrate 2;
(3) Mixing the filtrate 1 and the filtrate 2 and concentrating to obtain the traditional Chinese medicine composition.

In step (1), the ratio of the added water volume to the total mass of radix bupleuri, stir-fried fructus aurantii, radix rhapontici and fructus forsythiae is 6:1 mL/g;

In step (2), the ratio of the added water volume to the total mass of residue 1, radix scutellariae, poria cum radix pini and radix angelicae sinensis is 5:1 mL/g.

### Example 2 A traditional Chinese medicine composition for treating herpes zoster and preparation method thereof

The Chinese medicine composition includes the following components by weight: 20 portions of radix bupleuri, 50 portions of radix scutellariae, 20 portions of rhizoma pinelliae preparata, 50 portions of radix paeoniae rubra, 40 portions of stir-fried fructus aurantii, 30 portions of radix rhapontici, 30 portions of fructus forsythiae, 70 portions of poria cum radix pini, 30 portions of radix angelicae sinensis and 60 portions of rhizoma zingiberis recens.

### Preparation method:

(1) Weighing a prescribed amount of radix bupleuri, stir-fried fructus aurantii, radix rhapontici and fructus forsythiae, adding water and soaking 40 minutes, then boiling 50 minutes and 2 times to obtain a residue 1 and a filtrate 1;
(2) Mixing the residue 1 with radix scutellariae, poria cum radix pini and radix angelicae sinensis, adding water, boiling 1.5 hours and 2 times to obtain a filtrate 2;
(3) Mixing the filtrate 1 and the filtrate 2 and concentrating to obtain the traditional Chinese medicine composition.

in the above step (1), the ratio of the added water volume to the total mass of radix bupleuri, stir-fried fructus aurantii, radix rhapontici and fructus forsythiae is 10:1 mL/g.
in the above step (2), the ratio of the added water volume to the total mass of residue 1, radix scutellariae, poria cum radix pini and radix angelicae sinensis is 8:1 mL/g.

### Example 3 Atraditional Chinese medicine composition for treating herpes zoster andpreparation method thereof

The Chinese medicine composition includes the following components by weight: 25 portions of radix bupleuri, 35 portions of radix scutellariae, 12 portions of rhizoma pinelliae preparata, 35 portions of radix paeoniae rubra, 25 portions of stir-fried fructus aurantii, 40 portions of radix rhapontici, 40 portions of fructus forsythiae, 55 portions of poria cum radix pini, 15 portions of radix angelicae sinensis and 45 portions of rhizoma zingiberis recens.

### Preparation method:

(1) Weighing a prescribed amount of radix bupleuri, stir-fried fructus aurantii, radix rhapontici and fructus forsythiae, adding water and soaking for 30 minutes, boiling 40 minutes and 2 times to obtain a residue 1 and a filtrate 1;
(2) Mixing the residue 1 with radix scutellariae, poria cum radix pini and radix angelicae sinensis, adding water and the filtrate 1, boiling 1.5 hours and 2 times to obtain a filtrate 2;
(3) Mixing the filtrate 1 and the filtrate 2 and concentrating to obtain the traditional Chinese medicine composition.

In the above step (1), the ratio of the added water volume to the total mass of radix bupleuri, stir-fried fructus aurantii, radix rhapontici and fructus forsythiae is 8:1 mL/g; in the above step (2), the ratio of the added water volume to the total mass of residue 1, radix scutellariae, poria cum radix pini and radix angelicae sinensis is 6:1 mL/g; the volume ratio of the filtrate 1 to the added water is 1:10.

### Example 4 A traditional Chinese medicine composition for treating herpes zoster and preparation method thereof

The Chinese medicine composition includes the following components by weight: 40 portions of radix bupleuri, 45 portions of radix scutellariae, 18 portions of rhizoma pinelliae preparata, 45 portions of radix paeoniae rubra, 20 portions of stir-fried fructus aurantii, 35 portions of radix rhapontici, 35 portions of fructus forsythiae, 65 portions of poria cum radix pini, 25 portions of radix angelicae sinensis and 55 portions of rhizoma zingiberis recens.

### Preparation method:

(1) Weighing a prescribed amount of radix bupleuri, stir-fried fructus aurantii, radix rhapontici and fructus forsythiae, adding water and soaking for 30 minutes, boiling 40 minutes and 2 times to obtain a residue 1 and a filtrate 1;
(2) Mixing the residue 1 with radix scutellariae, poria cum radix pini and radix angelicae sinensis, adding water and the filtrate 1, boiling 1.5 hours and 2 times to obtain a filtrate 2;
(3) Mixing the filtrate 1 and the filtrate 2 and concentrating to obtain the traditional Chinese medicine composition.

In the above step (1), the ratio of the added water volume to the mass ratio of radix bupleuri, stir-fried fructus aurantii, radix rhapontici and fructus forsythiae is 8:1 mL/g; in the above step (2), the ratio of the added water volume to the total mass of residue 1, radix scutellariae, poria cum radix pini and radix angelicae sinensis is 6:1 mL/g; the volume ratio of the filtrate 1 to the added water is 3:10.

### Example 5 A traditional Chinese medicine composition for treating herpes zoster and preparation method thereof

The Chinese medicine composition includes the following components by weight: 30 portions of radix bupleuri, 40 portions of radix scutellariae, 15 portions of rhizoma pinelliae preparata, 40 portions of radix paeoniae rubra, 30 portions of stir-fried fructus aurantii, 30 portions of radix rhapontici, 30 portions of fructus forsythiae, 60 portions of poria cum radix pini, 20 portions of radix angelicae sinensis and 40 portions of rhizoma zingiberis recens.

### Preparation method:

(1) Weighing a prescribed amount of radix bupleuri, stir-fried fructus aurantii, radix rhapontici and fructus forsythiae, adding water and soaking for 30 minutes, boiling 40 minutes and 2 times to obtain a residue 1 and a filtrate 1;
(2) Mixing the residue 1 with radix scutellariae, poria cum radix pini and radix angelicae sinensis, adding water and the filtrate 1, boiling 1.5 hours and 2 times to obtain a filtrate 2;
(3) Mixing the filtrate 1 and the filtrate 2 and concentrating to obtain the traditional Chinese medicine composition.

In the above step (1), the ratio of the added water volume to the total mass of radix bupleuri, stir-fried fructus aurantii, radix rhapontici and fructus forsythiae is 8:1 mL/g; in the above step (2), the ratio of the added water volume to the total mass of residue 1, radix scutellariae, poria cum radix pini and radix angelicae sinensis is 6:1 mL/g; the volume ratio of the filtrate 1 to the added water is 2:10.

### Comparative Example 1

**The difference from Example 5 is** that the weight ratio of radix bupleuri, stir-fried fructus aurantii, radix rhapontici and fructus forsythiae is 1:1:3:3, i.e., 15 portions of radix bupleuri, 15 portions of stir-fried fructus aurantii, 45 portions of radix rhapontici and 45 portions of fructus forsythiae, and the other operations and steps are the same as those in Example 5.

### Comparative Example 2

**The difference from Example 5 is** that the weight ratio of radix bupleuri, stir-fried fructus aurantii, radix rhapontici and fructus forsythiae is 3:3: 1: 1, i.e., 45 portions of radix bupleuri, 45 portions of stir-fried fructus aurantii, 15 portions of radix rhapontici and 15 portions of fructus forsythiae, and the other operations and steps are the same as those in Example 5.

### Comparative Example 3

**The difference from Example 5 is** that, the weight ratio of radix scutellariae, poria cum radix pini and radix angelicae sinensis is 1:1:3, i.e. 24 portions of radix scutellariae, 24 portions of poria cum radix pini and 72 portions of radix angelicae sinensis, and the other operations and steps are the same as those in Example 5.

### Comparative Example 4

**The difference from Example 5 is** that, the weight ratio of radix scutellariae, poria cum radix pini and radix angelicae sinensis is 0.5:1:1, i.e. 24 portions of radix scutellariae, 24 portions of poria cum radix pini and 48 portions of radix angelicae sinensis, and the other operations and steps are the same as those in Example 5.

The following clinical trials are used to further illustrate the therapeutic effects of the traditional Chinese medicine composition of the present disclosure:
The liquid medicines prepared in Examples 1-5 and Comparative Examples 1-4 were packed into bags, with 200 mL in each bag. Treatment method: oral administration, one dose per day, 7 days as a course of treatment, take 1 course of treatment;
The efficacy standards refer to the relevant efficacy standards in the "Standards for Determining the Efficacy of traditional Chinese medicine Diseases".
Cure: all clinical symptoms disappear and laboratory tests are normal;
Markedly effective: clinical symptoms basically disappear, and relevant indicators of laboratory examinations were close to normal levels.
Effective: clinical symptoms were alleviated, relevant indicators of laboratory examinations were improved, and physical signs were improved.
Ineffective: clinical symptoms did not improve or worsen significantly.
The efficacy standards for this application are:
   Cure: Herpes zoster and skin lesions disappeared;
   Significant effective: herpes zoster basically disappeared, with slight pain and scattered scabs on the skin;
   Effective: a small amount of herpes, local pain;
   Ineffective: no improvement in herpes zoster and skin lesions.

The applicant has received 270 patients from 2016 to date, including 108 males and 162 females, aged between 35 and 85 years old. They were divided into 9 groups, with 30 patients in each group. The clinical experimental observation results are shown in Table 1.

**Table 1 Results of clinical trials on the treatment of herpes zoster**

| | Number of people | Cure | Cure rate% | Significant effect | Effective | Ineffective | Total effective rate% |
|---|---|---|---|---|---|---|---|
| Example 1: | 30 | 12 | 40% | 8 | 9 | 1 | 96.7% |
| Example 2: | 30 | 14 | 46.7% | 8 | 6 | 2 | 93.3% |
| Example 3: | 30 | 15 | 50% | 12 | 3 | 0 | 100% |
| Example 4: | 30 | 18 | 60% | 8 | 4 | 0 | 100% |
| Example 5: | 30 | 20 | 66.7% | 10 | 0 | 0 | 100% |
| Comparative Example 1 | 30 | 5 | 16.7% | 8 | 12 | 5 | 83.3% |
| Comparative Example 2 | 30 | 8 | 26.7% | 9 | 10 | 3 | 90% |
| Comparative Example 3 | 30 | 6 | 20% | 10 | 10 | 4 | 86.7% |
| Comparative Example 4 | 30 | 9 | 30% | 8 | 9 | 4 | 86.7% |

The experimental results show that the traditional Chinese medicine compositions produced in Examples 1-5 have good therapeutic effects on herpes zoster, with cure rates all above 40% and effective rates above 90%. In particular, in Example 5, by reasonably controlling the weight ratio of various ingredients, the obtained medicine composition has the best therapeutic effect, with a cure rate of 66.7% and an effective rate of 100%. In Comparative Examples 1-4, changing the weight ratio of several drug components out of the scope of protection of this application affect the therapeutic effects of the traditional Chinese medicine composition in a certain extent, especially the cure rate is significantly reduced, and the effective rate is also significantly lower than that of the Examples. This indicates that the traditional Chinese medicine composition of the present disclosure has a better effect on the treatment of herpes zoster by adopting a specific combination and dosage of raw herbs, which indicates that the traditional Chinese medicine composition of the present disclosure has an obvious effect on the treatment of herpes zoster.

### Typical cases:

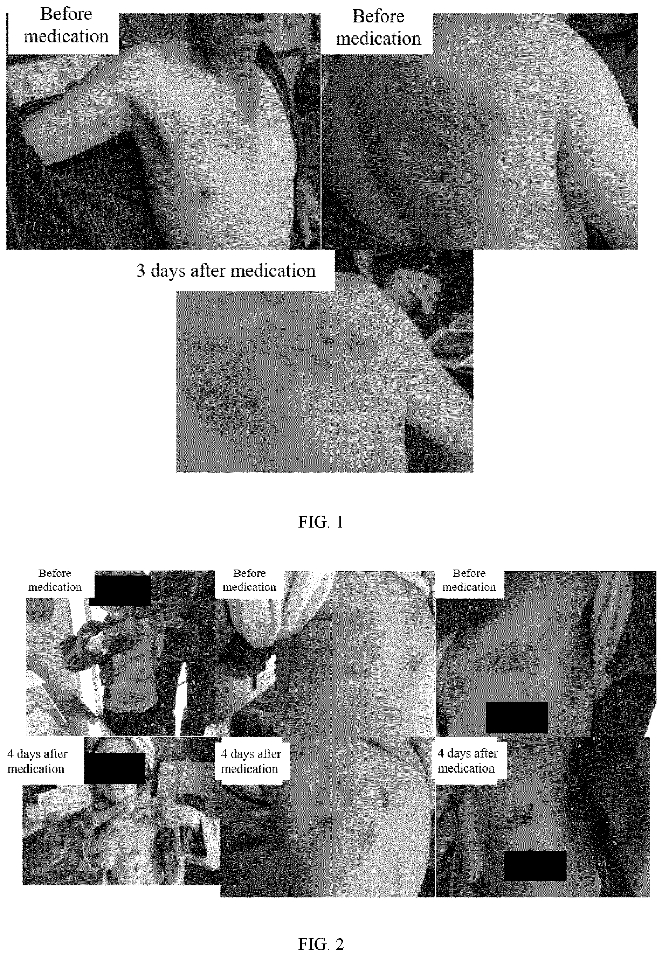

Case 1: the patient was a 65-year-old male. Four days before treatment, the patient experienced a right chest and ribs pain (mainly under the right armpit) without any obvious cause. The pain was mild and stabbing, and the patient did not pay special attention. One day before treatment, the patient developed band-like, clustered blisters on the right side of the chest and ribs with needle-like pain. The pain was aggravated by friction with clothing, and the patient was irritable, so the patient sought medical attention. After 3 days of treatment with the traditional Chinese medicine composition disclosed in Example 5, the right ribs pain of the patient disappeared and the patient felt normal; the skin lesion area was significantly reduced and most of the herpes scabs formed. The effect diagram before and after treatment is shown in FIG. 1.

Case 2: the patient was an 83-year-old female. The patient experienced burning and stinging pain in her left upper back, left forearm, left chest, and left armpit 8 days before treatment. Clustered blisters appeared 6 days before treatment, and the pain worsened and quickly turned into blisters. The patient used acyclovir ointment but felt no improvement. The symptoms of the patient worsened 2 days before treatment, and the patient could not sleep at night. The patient reported suicidal thoughts, so the patient was presented to hospital by her family. After taking 3 sets of the traditional Chinese medicine composition disclosed in the above Example 5, the symptoms almost disappeared. After taking another 3 sets of the traditional Chinese medicine composition for consolidation, the patient did not have any sequelae. The effect diagram before and after treatment is shown in FIG. 2.

## Claims

1. A traditional Chinese medicine composition for treating herpes zoster, **characterized in that**, the traditional Chinese medicine composition comprises the following components: radix bupleuri, radix scutellariae, rhizoma pinelliae preparata, radix paeoniae rubra, stir-fried fructus aurantii, radix rhapontici, fructus forsythiae, poria cum radix pini, radix angelicae sinensis and rhizoma zingiberis recens.

2. The traditional Chinese medicine composition according to claim 1, **characterized in that**, the weight ratio of the radix bupleuri, stir-fried fructus aurantii, radix rhapontici and fructus forsythiae is 0.5-2:0.5-2:1:1.

3. The traditional Chinese medicine composition according to claim 2, **characterized in that**, the weight ratio of the radix bupleuri, stir-fried fructus aurantii, radix rhapontici and fructus forsythiae is 1-2:0.5-2:1:1.

4. The traditional Chinese medicine composition according to claim 3, **characterized in that**, the weight ratio of the radix bupleuri, stir-fried fructus aurantii, radix rhapontici and fructus forsythiae is 1:1:1:1.

5. The traditional Chinese medicine composition according to claim 1, **characterized in that**, the weight ratio of the radix scutellariae, poria cum radix pini and radix angelicae sinensis is 1-5:2-7:1.

6. The traditional Chinese medicine composition according to claim 5, **characterized in that**, the weight ratio of the radix scutellariae, poria cum radix pini and radix angelicae sinensis is 2-4:3-5:1.

7. The traditional Chinese medicine composition according to claim 6, **characterized in that**, the weight ratio of the radix scutellariae, poria cum radix pini and radix angelicae sinensis is 2:3:1.

8. The traditional Chinese medicine composition according to claim 1, **characterized in that**, the traditional Chinese medicine composition comprises the following components according to portions by weight: 20-40 portions of radix bupleuri, 30-50 portions of radix scutellariae, 10-20 portions of rhizoma pinelliae preparata, 30-50 portions of radix paeoniae rubra, 20-40 portions of stir-fried fructus aurantii, 20-40 portions of radix rhapontici, 20-40 portions of fructus forsythiae, 50-70 portions of poria cum radix pini, 10-30 portions of radix angelicae sinensis and 40-60 portions of rhizoma zingiberis recens.

9. The traditional Chinese medicine composition according to claim 8, **characterized in that**, the traditional Chinese medicine composition comprises the following components according to portions by weight: 30 portions of radix bupleuri, 40 portions of radix scutellariae, 15 portions of rhizoma pinelliae preparata, 40 portions of radix paeoniae rubra, 30 portions of stir-fried fructus aurantii, 30 portions of radix rhapontici, 30 portions of fructus forsythiae, 60 portions of poria cum radix pini, 20 portions of radix angelicae sinensis and 40 portions of rhizoma zingiberis recens.

10. A preparation method for the traditional Chinese medicine composition according to any one of claims 1 to 9, comprising the following steps:
(1) Weighing a prescribed amount of radix bupleuri, stir-fried fructus aurantii, radix rhapontici and fructus forsythiae, adding water and boiling to obtain a residue 1 and a filtrate 1;
(2) Mixing the residue 1 with the radix scutellariae, poria cum radix pini and radix angelicae sinensis, adding water and boiling to obtain a filtrate 2;
(3) Mixing the filtrate 1 and the filtrate 2 and concentrating to obtain the traditional Chinese medicine composition.

11. The preparation method according to claim 10, **characterized in that**, before boiling in step (1), the herbs are soaked in water for 20-40 minutes; the boiling time is 30-50 minutes; the boiling is repeated 1-2 times; the ratio of the added water volume to the total mass of radix bupleuri, stir-fried fructus aurantii, radix rhapontici and fructus forsythiae is 6-10:1 mL/g.

12. The preparation method according to claim 10, **characterized in that**, the boiling time in step (2) is 1-1.5h; the boiling is repeated 1-2 times; the ratio of the added water volume to the total mass of residue 1, radix scutellariae, poria cum radix pini and radix angelicae sinensis is 5-8:1 mL/g.

13. The preparation method according to claim 10, **characterized in that**, the filtrate 1 is added in step (2), and the volume ratio of the filtrate 1 to the added water is 1-3: 10.

14. Use of the traditional Chinese medicine composition according to any one of claims 1 to 9 in the production of a drug for treating herpes zoster.
